# EUROPEAN PATENT APPLICATION

(11) **EP 1 433 426 A1**
(43) Date of publication of application: **30.06.2004**
(21) Application number: 02380271.3
(22) Date of filing: 24.12.2002
(51) Int. Cl.: A61B 17/70

(54) **Posterior vertebral body fastening device in the lumbar region**

(71) Applicant: JB Spine Instruments, S.l., 46009 Valencia (ES)
(72) Inventor: Barbera Tomas, David, 46009 Valencia (ES); Barbera Alacreu, José Vicente, 46009 Valencia (ES)
(74) Representative: Isern-Cuyas, Maria Luisa

(57) **Abstract**

The posterior vertebral body fastening device in the lumbar region is made up of:
- Two identical plates (1) with two end orifices (2) in each one
- Four vertebral screws (3) the heads of which have flexibility grooves (6) and a conical cavity (10) for inserting a blocking screw (4) the head of which has the same conical shape (14) as the cavity (10)

Locking of the screws (3) on the plate (1) can be made by screwing their threaded heads (15) on to the screwed orifices (16) of the plate or by lids (17) or by conical matching of the heads (19) to the orifices (18).

Transmission of axial stresses to the graft or intersomatic box (22) is facilitated by replacing the plate orifices (2) by a groove (21); or else by a reduction (23) in the upper part of the screws (3) that permits pivoting (24).

## Description

### PURPOSE

The purpose to which the invention protected by this Patent refers consists of a "Posterior vertebral body fastening device in the lumbar region".

This is made up of a set of mechanical means the structure of which has been designed with an arrangement that permits, by means of fastening some of its component elements with screws to the vertebra, obtaining stabilization of a vertebral level of the lumbar column.

### HISTORY

The screwed posterior fastenings presently used on the lumbar column are transpedicular. The screws penetrate the two pedancles, made up of a type of osseous cylinders beginning at the back part of the vertebral body to surround and protect the spinal cord.

The transpedicular fastenings presently constitute optimum performance of the instrumentation for the vertebral column and, therefore, are used in almost all surgical techniques, including those in which it would be more suitable to consider another type of fastening.

The Posterior Lumbar Interbody Fusion (PLIF) consists in the bone fusion of two adjoining vertebral bodies through the intermediate space between them, known as the discal space as it is occupied by the intervertebral disc in normal conditions. Usually a bone graft has been used to occupy this space and favor the fusion, although in the last years the use of prefabricated boxes to increase the resistance of this type of graft has grown, whilst the use of transpedicular fastening is generalized when only grafts are made.

The use of grafts of prefabricated boxes in PLIF is accompanied by a posterior fastening using transpedicular screws.

The PLIF is an operation during which numerous anatomic structures are removed: the articular apophyses, one or more laminas and in some cases part of the spine apophyses. Therefore, it would seen logical to take advantage of the removal of these structures to apply the necessary posterior fastening. But the transpedicular fastening is made through the pedancles, precisely one of the few structures the PLIF keeps intact. In other words, an operation that is difficult in itself, meaning that the manipulating and removal of numerous anatomic structures have to be completed by another completely different operation which, in turn, manipulates other anatomic sectors. This does not seem to be a suitable way of complying with the "minimum invasion" principle recommended in any other operation.

The transpedicular technique includes the placing of bars joining the screws, adjustment of a blocking system of the bars to the screws, placing connecting between bars... In short, a costly and long operation added to the operation of the PLIF.

The danger always associated to transpedicular fastening must be added to this: the screw that has to be "blindly" inserted through the narrow osseous cylinder that is the pedancle, helped by anatomic references not always the same in all individuals. Therefore, constant radiological verification of the path of the screw through the pedancle is necessary, as a deviation could cause serious damage to the spinal cord.

### DESCRIPTION OF THE INVENTION

The purpose of the invention object of this Patent consists in the elimination of the inconveniences proper of posterior fastenings existing to date and has been conceived and designed with this purpose in mind.

In accordance with this, the structure of said invention has been designed and includes the following elements as a minimum:
- Four vertebral screws to be inserted into the posterior part of the vertebral bodies of the instrumented level, on both side of the spinal cord
- Two identical plates through the orifices of which said screws will be inserted, in such a way that the plate between the two vertebral bodies transmits part of the stresses to which the discal space is submitted

The described structure corresponds to minimum efficiency, as the form and size of the elements can be varied, as well as the way of performing the coupling between plate and screws. It is even possible to place one plate only instead of two on each side if the surgeon considers this convenient. It is also possible the plate includes a PLIF intersomatic box in its structure instead of being placed separately.

Instead of using transpedicular screws for the required posterior fastening accompanying the intersomatic boxes or the graft, the application technique of the claimed device uses a plate and non-transpedicular screws. The plate is located on the posterior part and next to the vertebral bodies, underneath the nervous root corresponding to the instrumented level. This is reached after a laminectomy, which is obtained thanks to the manipulation of the spinal cord, an operation only possible in the lumbar region.

The screws are inserted into the plate orifices located on the posterior part of the vertebral bodies and, therefore, the insertion of transpedicular screws is not necessary. This plate uses the removal of the lamina, which has to be done during the PLIF operation, and needs no further manipulation or anatomic removals with the result its use is much simpler and shorter than the transpedicular screws. The posterior fastening function required by the PLIF is now no longer a costly and dangerous addition to the operation.

### BRIEF DESCRIPTION OF THE DRAWINGS

To complement the description of the invention and facilitate the interpretation of the formal, structural and functional characteristics of its purpose, attached are drawings in which different aspects of a preferred performance of the "Anterior cervical fastening device", constituting the purpose of this Patent, are schematically represented.

In said drawings:

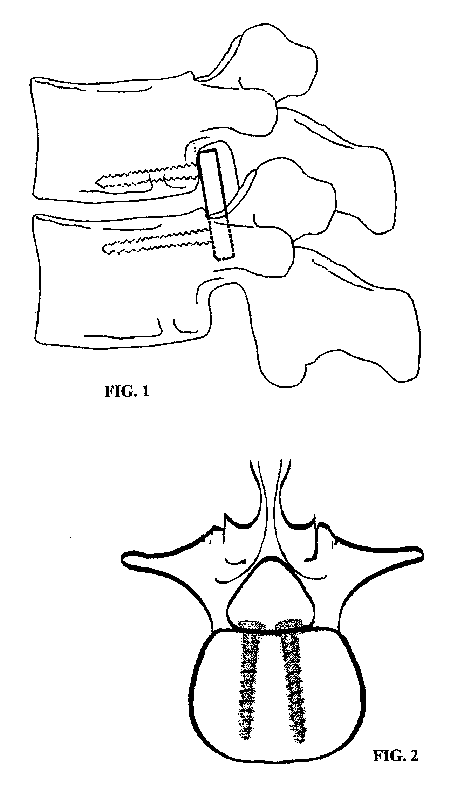

- Figure 1 is a longitudinal semi-section showing the place where the implantation in the lumbar column is made
- Figure 2 is a transversal section showing the place where the implantation in the lumbar column is made

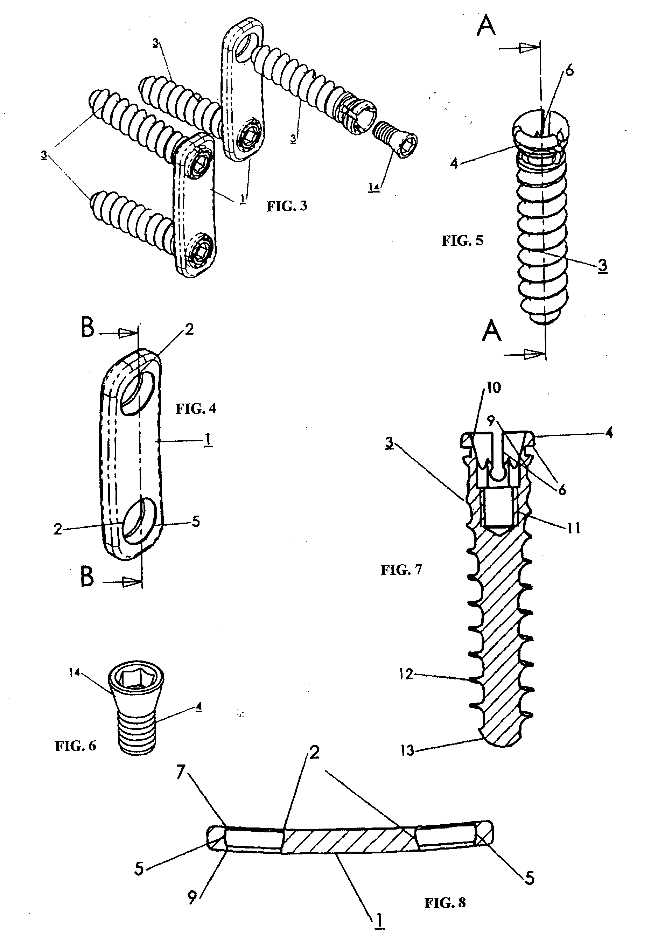

- Figure 3 represents a perspective view of the overall performance of the device
- Figure 4 represents a perspective view of a performance of the plate
- Figure 5 is a perspective view of a performance of the vertebral screw
- Figure 6 is a perspective view of a performance of the blocking screw
- Figure 7 is a longitudinal section of a performance of the vertebral screw cut by a plane the design of which is represented in Figure 5 as A-A
- Figure 8 is a longitudinal section of a performance of the plate cut by a plane the design of which is represented in Figure 4 as B-B
- Figure 9 is a perspective view of the partial performance of the device

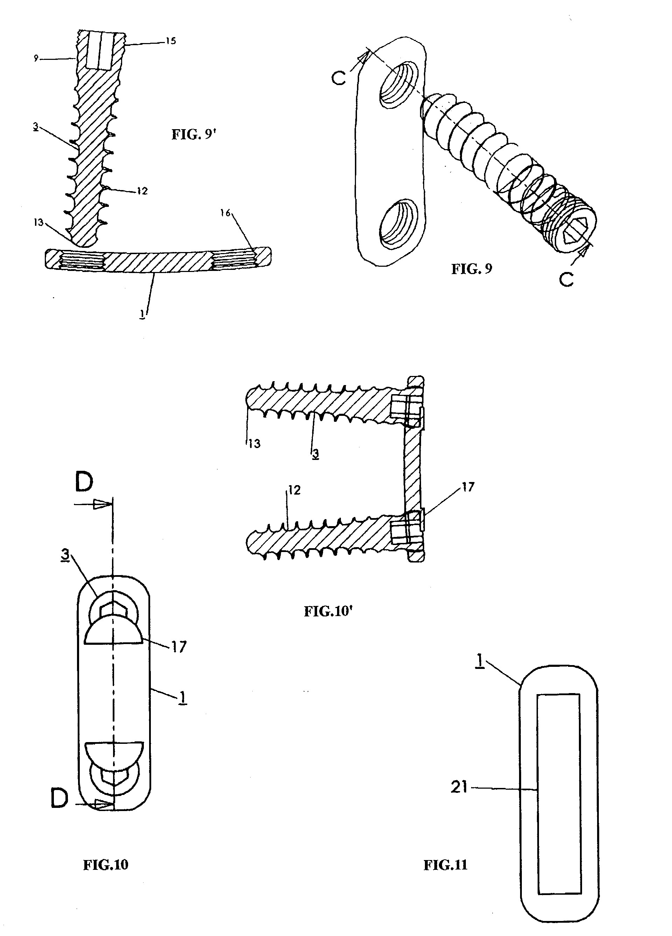

- Figure 9' is a longitudinal section of a partial performance of the device cut by a plane the design of which is represented in Figure 9 as C-C
- Figure 10 is a front elevation of a variant of the overall device performance
- Figure 10' is a longitudinal section of the device performance cut by a plane the design of which is represented in Figure 10 as D-D

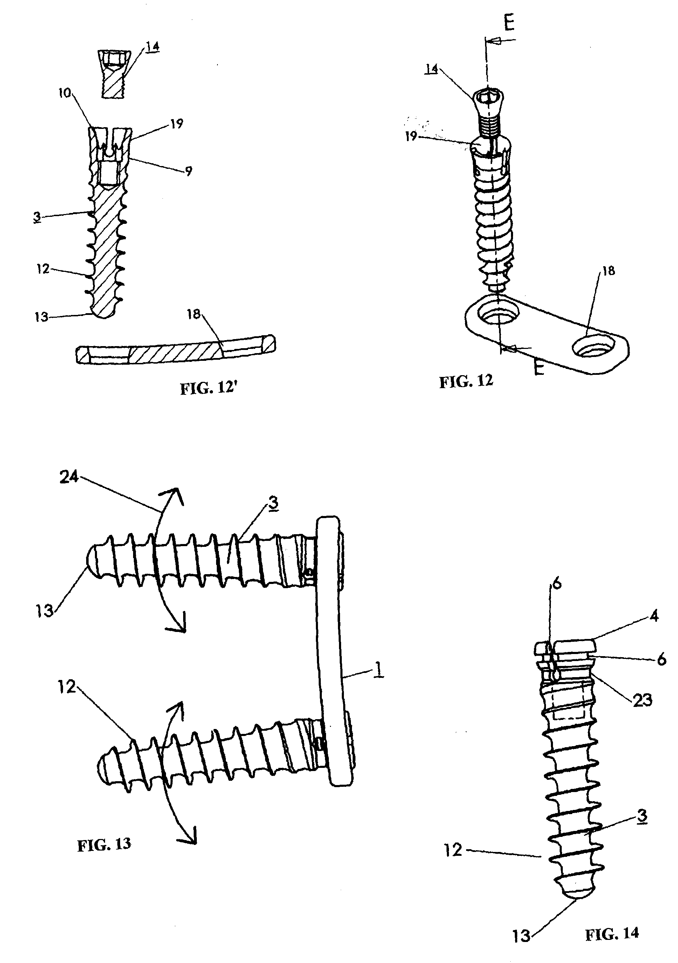

- Figure 11 is a front elevation of a variant of the plate performance
- Figure 12 represents a perspective view of the overall device performance
- Figure 12' is a longitudinal section of the device performance cut by a plane the design of which is represented in Figure 12 as E-E
- Figure 13 represents a side elevation of the overall device performance
- Figure 14 is a front elevation of the vertebral screw performance

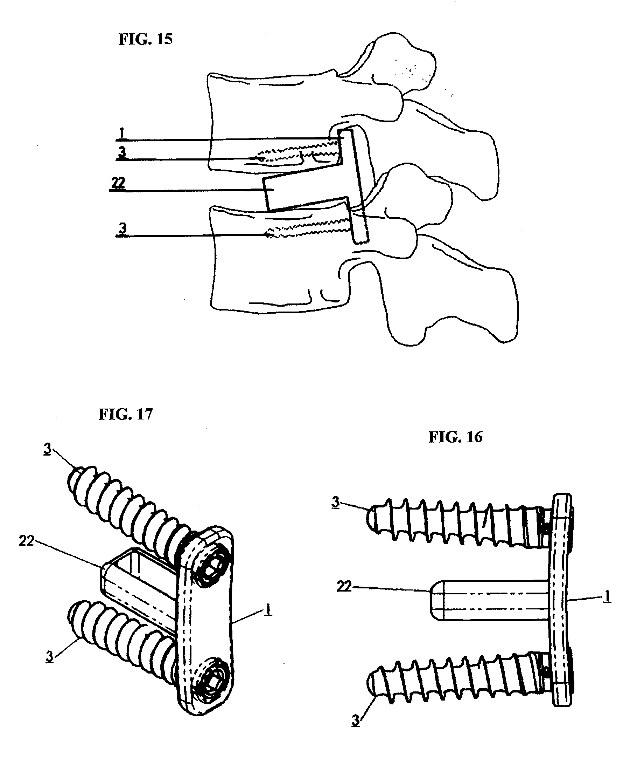

- Figure 15 is a longitudinal section showing the place where a performance of the lumbar column implantation is positioned
- Figure 16 represents a side view of the overall device performance with the intersomatic box
- Figure 17 represents a perspective view of the overall device performance with the box shown in Figure 16

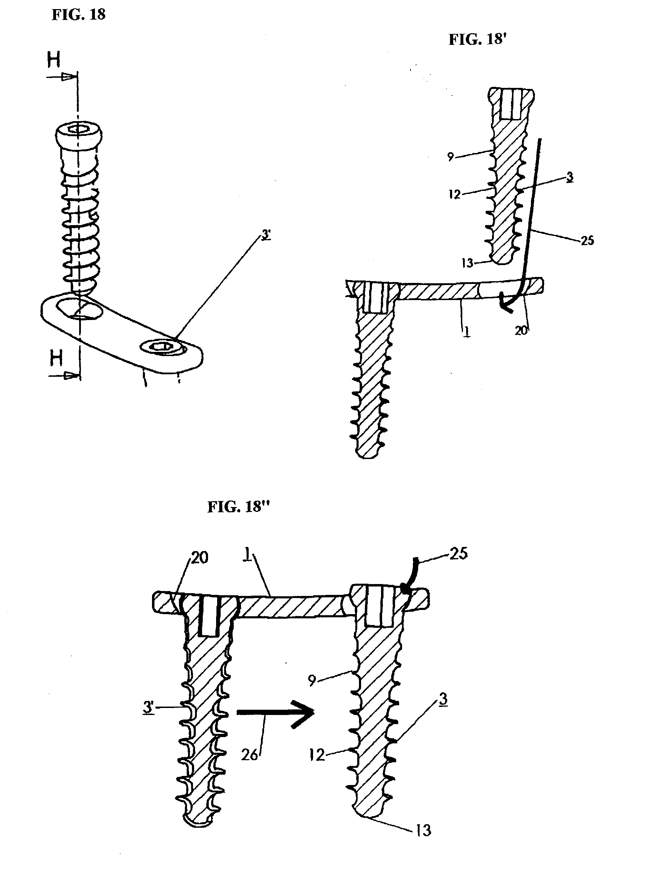

- Figure 18 is a perspective view of the overall device performance variant
- Figure 18' is a longitudinal section showing the insertion path of the vertebral screw during the device performance in Figure 18, cut by a plane the design of which is represented as H-H in said Figure 18
- Figure 18" is a longitudinal section showing the insertion path of the vertebral screw during the device performance of Figure 18, cut by a plane the design of which is represented in Figure 18 as H-H

### DESCRIPTION OF A PREFERRED PERFORMANCE

To clearly show the nature and scope of the advantageous application of the "Posterior vertebral body fastening device in the lumbar region", purpose of the claimed invention, the following is a description of its structure and the characteristics of its component elements, making reference to the drawings which, on representing a preferred performance of said purpose for information, must be considered in the widest sense and not limiting of the application and content of the claimed invention.

The minimum efficient structure of the "Posterior vertebral body fastening device in the lumbar region" is composed of:
- Two identical plates (1) with two end orifices (2) each one made up of a spherical cavity (5) and two drill holes (7)-(9) with different diameters
- Four vertebral screws (3), two for each plate, the heads of which have a spherical shape (4) to adjust to the cavities (5) of the plate orifices and grooves (6) that make the head flexible so that it is possible to insert it into the plate orifice, in spite of the fact its diameter is slightly larger than the diameter of the upper drill hole (7) of the orifice. Meanwhile, before reaching the thread, the screw has a cylindrical part (8) that adjusts to the lower drill hole (9) of the plate orifice preventing it from moving inside the latter and the head of the screws has a conical cavity (10) ending up in a screwed drill hole (11) to insert both blocking screws. The screw spire (12) has the necessary depth to permit sufficient fastening to the spongy bone with a Roman and non self-screwing point (13) so that it cannot pass through the anterior cortical surface of the vertebral body.
- Four blocking screws (4), one for each vertebral screw (3), screwed into the cavity of their heads. The head of the blocking screws has a conical shape (14) to press on to the cavity walls (10) of the vertebral screw head whilst being inserted and thus increase its diameter thanks to the grooves (6) that make said head flexible so that it becomes blocked in the plate orifice (2)

Setting of the crews (3) in the plate (1) can be done in various ways, such as: screwing the head (15) on to one of the two screwed orifices (16) of the plate (see Figures 9 and 9'); the use of lids (17) that prevent the screw from leaving its housing (see Figures 10 and 10'); or else trunk-conical shaping of the plate orifices (18) and the heads (19) of the vertebral screws, with the same conical shape (see Figures 12 and 12').

Transmission of axial stresses to the graft or intersomatic box carried out by the PLIF can be made easier by replacing the orifices (2) of the plate (1) with a groove (21) (see Figure 11) that permits vertical movement of the screws; or else by a reduction (23) in the upper part of the vertebral screws that permits nodding (24) of the screws (3) inside their respective housings in the plates (1) (see Figures 13 and 14).

The device can include a self-compressing system (20) as, when the vertebral screws (3) are inserted into the orifices in which they are housed, the discal space is compressed and, therefore, the graft or the intersomatic box interposed therein (see Figures 18, 18' and 18").

The device can include a prefabricated PLIF intersomatic box (22), located in the center of the plate (1) so that the whole set is inserted in the same operation instead of placing the prefabricated box first and then the plate (1) and screws (3) on the instrumented vertebral bodies (see Figures 15, 16 and 17).

According to the above, the basic structure of the device admits slight variants as regards certain partial aspects. These do not change or impair its essentiality and thus constitute "a group of interrelated inventions resulting in a unique general inventive concept". Therefore, in view of Article 24.1 of the Patent Law, they are included in this request.

## Claims

1. Posterior vertebral body fastening device in the lumbar region **characterized by** the fact its minimum efficient structure includes the following elements:
- Two identical plates (1) with two end orifices (2) each one, made up of a spherical cavity (5) and two drill holes (7)-(9) with different diameters
- Four vertebral screws (3), two for each plate, the heads of which have a spherical shape (4) to adjust to the cavities (5) of the plate orifices and grooves (6) that make the head flexible so that it is possible to insert it into the plate orifice, in spite of the fact its diameter is slightly larger than the diameter of the upper drill hole (7) of the orifice. Meanwhile, before reaching the thread, the screw has a cylindrical part (8) that adjusts to the lower drill hole (9) of the plate orifice preventing it from moving inside the latter and the head of the screws has a conical cavity (10) ending up in a screwed drill hole (11) to insert both blocking screws. The screw spire (12) has the necessary depth to permit sufficient fastening to the spongy bone with a Roman and non self-screwing point (13) so that it cannot pass through the anterior cortical surface of the vertebral body.
- Four blocking screws (4), one for each vertebral screw (3), screwed into the cavity of their heads. The head of the blocking screws has a conical shape (14) to press on to the cavity walls (10) of the vertebral screw head whilst being inserted and thus increase its diameter thanks to the grooves (6) that make said head flexible so that it becomes blocked in the plate orifice (2)

2. Posterior vertebral body fastening device in the lumbar region, according to claim 1, **characterized by** the fact the housing of the screws (3) on the plate (1) can be made in various ways, such as: screwing the head (15) on to one of the two screwed orifices (16) of the plate (see Figures 9 and 9'); the use of lids (17) that prevent the screw from leaving its housing (see Figures 10 and 10'); or else trunk-conical shaping of the plate orifices (18) and the heads (19) of the vertebral screws, with the same conical shape (see Figures 12 and 12').

3. Posterior vertebral body fastening device in the lumbar region, according to the above claims, **characterized by** the fact transmission of axial stresses to the graft or intersomatic box carried out by the PLIF can be made easier by replacing the orifices (2) of the plate (1) with a groove (21) (see Figure 11) that permits vertical movement of the screws; or else by a reduction (23) in the upper part of the vertebral screws that permits nodding (24) of the screws (3) inside their respective housings in the plates (1) (see Figures 13 and 14).

4. Posterior vertebral body fastening device in the lumbar region, according to the above claims, **characterized by** the fact it includes a self-compressing system (20) as, when the vertebral screws (3) are inserted into the orifices in which they are housed, the discal space is compressed and, therefore, the graft or the intersomatic box interposed therein (see Figures 18, 18' and 18").

5. Posterior vertebral body fastening device in the lumbar region, according to the above claims, **characterized by** the fact it can include a prefabricated PLIF intersomatic box (22), located in the center of the plate (1) so that the whole set is inserted in the same operation instead of placing the prefabricated box first and then the plate (1) and screws (3) on the instrumented vertebral bodies (see Figures 15, 16 and 17).
